(19) Europäisches Patentamt
European Patent Office
Office européen des brevets

(11) Veröffentlichungsnummer: **0 004 288**
**A2**

# (12) EUROPÄISCHE PATENTANMELDUNG

(21) Anmeldenummer: **79100535.8**

(22) Anmeldetag: **23.02.79**

(51) Int. Cl.²: **C 07 D 211/22**, C 07 D 207/08, C 07 D 223/08, A 61 K 31/40, A 61 K 31/445, A 61 K 31/55

(30) Priorität: **18.03.78 DE 2811952**

(43) Veröffentlichungstag der Anmeldung: **03.10.79 Patentblatt 79/20**

(84) Benannte Vertragsstaaten: **BE CH DE FR GB IT NL SE**

(71) Anmelder: **Merck Patent Gesellschaft mit beschränkter Haftung, Frankfurter Strasse 250, D-6100 Darmstadt (DE)**

(72) Erfinder: **Uhl, Jürgen, Dr., Odenwaldstrasse 49, D-6104 Seeheim 1 (DE)**
Erfinder: **Marx, Dieter, Dr., Mainzer Strasse 14, D-6108 Weiterstadt 1 (DE)**
Erfinder: **Hausberg, Hans-Heinrich, Dr., Hahlgartenstrasse 12a, D-6108 Weiterstadt 1 (DE)**
Erfinder: **Strehlow, Wighard, Dr., Ahornweg 12, D-6102 Pfungstadt 2 (DE)**
Erfinder: **Minck, Klaus-Otto, Dr., Büchestrasse 8, D-6105 Ober-Ramstadt (DE)**
Erfinder: **Müller-Calgan, Helmut, Dr., Peter-Behrens-Strasse 20, D-6100 Darmstadt (DE)**
Erfinder: **Seyfried, Christoph, Dr., Am Landbach 9, D-6104 Seeheim (DE)**

(54) **Phenoxyalkylamine, diese enthaltende pharmazeutische Zubereitungen und Verfahren zu ihrer Herstellung.**

(57) Phenoxyalkylamine der allgemeinen Formel I

Ar–O–E I

worin

Ar Phenyl oder ein- oder zweifach durch F, Cl, Br, Alkyl oder Alkoxy mit jeweils 1–4 C-Atomen, Cycloalkyloxy mit 3–6 C-Atomen, $CF_3$, CN, Alkylthio mit 1–4 C-Atomen, $SCF_3$, OH und/oder Alkanoyloxy mit 1–10 C-Atomen substituiertes Phenyl,

R (1-R¹ -2-pyrrolidyl)–$CH_2$–$CHR^2$–, (1-R¹ -2-piperidyl)–$CH_2$–$CHR^2$– oder 1-R¹ -3-Z-4-hexahydroazepinyl,

$R^1$ H, Alkyl oder Alkenyl mit jeweils bis zu 4-C-Atomen, Cyclopropylmethyl oder Benzyl,

$R^2$ H, Alkyl mit 1–4 C-Atomen oder Phenyl und

Z Alkyl mit 1–4 C-Atomen

bedeuten, worin jedoch Ar nur dann eine p-Fluorphenylgruppe bedeutet, wenn nicht gleichzeitig R eine 2-(1-Methyl-2-piperidyl)-äthylgruppe ist,

können z. B. hergestellt werden durch Umsetzung eines Phenols der Formel II

Ar–OH II

mit einer Verbindung der Formel III

X–R III

worin

X Cl, Br, J oder OH bedeutet.

Die neuen Verbindungen und ihre Säureadditionssalze können zur Herstellung von Arzneimitteln mit Wirkungen auf das Zentralnervensystem verwendet werden.

EP 0 004 288 A2

0004288

# Phenoxyalkylamine, diese enthaltende pharmazeutische Zubereitungen und Verfahren zu ihrer Herstellung

Die Erfindung betrifft neue Phenoxyalkylamine der allgemeinen Formel I

Ar-O-R I

worin

| | |
|---|---|
| Ar | Phenyl oder ein- oder zweifach durch F, Cl, Br, Alkyl oder Alkoxy mit jeweils 1 - 4 C-Atomen, Cycloalkoxy mit 3 - 6 C-Atomen, $CF_3$, CN, Alkylthio mit 1 - 4 C-Atomen, $SCF_3$, OH und/oder Alkanoyloxy mit 1 - 10 C-Atomen substituiertes Phenyl, |
| R | (1-$R^1$-2-pyrrolidyl)-$CH_2$-$CHR^2$-, (1-$R^1$-2-piperidyl)-$CH_2$-$CHR^2$- oder 1-$R^1$-3-Z-4-hexahydroazepinyl, |
| $R^1$ | H, Alkyl oder Alkenyl mit jeweils bis zu 4 C-Atomen, Cyclopropylmethyl oder Benzyl, |
| $R^2$ | H, Alkyl mit 1 - 4 C-Atomen oder Phenyl und |
| Z | Alkyl mit 1 - 4 C-Atomen |

bedeuten, worin jedoch Ar nur dann eine p-Fluorphenylgruppe bedeutet, wenn nicht gleichzeitig R eine 2-(1-Methyl-2-piperidyl)-äthylgruppe ist,

sowie ihre physiologisch unbedenklichen Säureadditionssalze.

Der Erfindung lag die Aufgabe zugrunde, neue Verbindungen aufzufinden, die zur Herstellung von Arzneimitteln verwendet werden können. Diese Aufgabe wurde durch die Bereitstellung der Verbindungen der Formel I gelöst.

Es wurde gefunden, daß die Verbindungen der Formel I und ihre physiologisch unbedenklichen Säureadditionssalze wertvolle pharmakologische Eigenschaften besitzen. So zeigen sie insbesondere Wirkungen auf das Zentralnervensystem, vor allem antidepressive Wirkungen. Im einzelnen wirken sie z.B. reserpinantagonistisch (feststellbar z. B. an Mäusen gegenüber Reserpin in Anlehnung an die Methode von Askew, Life Science, Bd. 10 (1963), Seiten 725 - 730), antikataleptisch (feststellbar z. B. an Ratten gegenüber Tetrabenazin in Anlehnung an die Methode von Giurgea et al., Medicina Experimentalis, Bd. 9, (1963), Seiten 249 - 262) und antiptotisch (feststellbar z. B. gegenüber Reserpin in Anlehnung an die Methodik von Domenjoz und Theobald, Arch. int. pharmacodyn., Bd. 120 (1959), Seite 450 ff., mit der Bewertung nach Rubin et al., J. Pharmacol. Exp. Therap., Bd. 120 (1957), Seiten 125 - 136), potenzieren die Wirkung des 5-Hydroxytryptophans bei Mäusen (Methode: ähnlich wie Ross et al., Acta pharmacol. et toxicol., Bd. 39 (1976), Seiten 152 - 166) und erhöhen und/oder verlängern die zentralen Auswirkungen einer Erregung und Temperatursteigerung, die D-Amphetaminsulfat (z.B. 1,5 mg/kg s.c., 1 Stunde nach der Prüfsubstanz verabfolgt, die ebenfalls s.c. appliziert wird) und Aggregation (Zusammensetzung von 5 Ratten in einem Glas) auslösen (Methodik nach Müller-Calgan et al. in Zippel, H.P. (Ed.): Memory and Transfer of Information, Plenum Press, New York - London, 1973, Seiten 87 - 125). Die Substanzen haben einen Einfluß auf die biogenen Amine des ZNS. So führen sie z.B. in vitro zur Aufnahmehemmung von Noradrenalin, 5-Hydroxytryptamin und Dopamin (Methodik: Kannengießer et al.,

Biochem. Pharmacol., Bd. 22 (1973), Seiten 73 - 84) in Synaptosomen und hemmen in vivo (Methodik: Carlsson et al., Europ. J. Pharmacol., Bd. 5 (1969), Seiten 357 - 366; 367 - 373) die durch Tyraminderivate induzierte Katecholamin- und Serotonin-Freisetzung im Gehirn.

Verbindungen der Formel I und ihre physiologisch unbedenklichen Säureadditionssalze können daher als Arzneimittelwirkstoffe und auch als Zwischenprodukte zur Herstellung anderer Arzneimittelwirkstoffe verwendet werden.

Gegenstand der Erfindung sind die Phenoxyalkylamine der Formel I sowie ihre physiologisch unbedenklichen Säureadditionssalze.

In den Resten Ar, $R^1$, $R^2$ und Z bedeutet Alkyl vorzugsweise Methyl, ferner auch Äthyl, n-Propyl, Isopropyl, n-Butyl, Isobutyl, sek.-Butyl oder tert.-Butyl. Alkoxy ist vorzugsweise Methoxy, ferner auch Äthoxy, n-Propoxy, Isopropoxy, n-Butoxy, Isobutoxy, sek.-Butoxy oder tert.-Butoxy. Cycloalkoxy ist vorzugsweise Cyclopentyloxy oder Cyclohexyloxy, ferner auch Cyclopropyloxy, Cyclobutyloxy, 1-, 2- oder 3-Methylcyclobutyloxy, 1-, 2- oder 3-Methylcyclopentyloxy. Alkylthio steht vorzugsweise für Methylthio, aber auch für Äthylthio, n-Propylthio, Isopropylthio, n-Butylthio, Isobutylthio, sek.-Butylthio oder tert.-Butylthio. Alkanoyloxy ist vorzugsweise unverzweigt und bedeutet bevorzugt Acetoxy, Propionyloxy, Butyryloxy, Valeryloxy, Hexanoyloxy, Heptanoyloxy, Octanoyloxy, Nonanoyloxy oder Decanoyloxy, ferner z.B. auch Formyloxy, Isobutyryloxy, Isovaleryloxy, Methyläthylacetoxy, Trimethylacetoxy, Isohexanoyloxy. Alkenyl ist vorzugsweise Allyl, ferner auch Vinyl, Propenyl, Isopropenyl, 1-Buten-1- oder -2-yl, 2-Buten-1- oder -2-yl, 3-Buten-1- oder -2-yl, 2-Methyl-1-propen-1- oder -2-yl oder 2-Methyl-2-propen-1-yl.

Falls Ar eine substituierte Phenylgruppe bedeutet, so ist diese vorzugsweise einfach substituiert. Sie kann jedoch auch zweifach substituiert sein, wobei die Substituenten gleich oder verschieden sein können. Bevorzugte Substituenten an der Phenylgruppe sind F, Cl, Br, Methyl, Methoxy, Cyclopentyloxy, Trifluormethyl und Methylthio. Im einzelnen ist Ar bevorzugt Phenyl, o-, m- oder p-Fluorphenyl, o-, m- oder p-Chlorphenyl, o-, m- oder p-Bromphenyl, o-, m- oder p-Tolyl, o-, m- oder p-Methoxyphenyl, o-, m- oder p-Cyclopentyloxyphenyl, o-, m- oder p-Trifluormethylphenyl, o-, m- oder p-Methylthiophenyl, ferner z.B. o-, m- oder p-Äthylphenyl, o-, m- oder p-n-Propylphenyl, o-, m- oder p-Isopropylphenyl, o-, m- oder p-n-Butylphenyl, o-, m- oder p-Isobutylphenyl, o-, m- oder p-Äthoxyphenyl, o-, m- oder p-n-Propoxyphenyl, o-, m- oder p-Isopropoxyphenyl, o-, m- oder p-n-Butoxyphenyl, o-, m- oder p-Isobutoxyphenyl, o-, m- oder p-Cyclopropyloxyphenyl, o-, m- oder p-Cyclohexyloxyphenyl, o-, m- oder p-Cyanphenyl, o-, m- oder p-Äthylthiophenyl, o-,m- oder p-n-Propylthiophenyl, o-, m- oder p-Isopropylthiophenyl, o-, m- oder p-n-Butylthiophenyl, o-, m- oder p-Isobutylthiophenyl, o-, m- oder p-Trifluormethylthiophenyl, o-, m- oder p-Hydroxyphenyl, o-, m- oder p-Acetoxyphenyl, o-, m- oder p-Propionyloxyphenyl, o-, m- oder p-Butyryloxyphenyl, o-, m- oder p-Isobutyryloxyphenyl, o-, m- oder p-Valeryloxyphenyl, o-, m- oder p-Hexanoyloxyphenyl, o-, m- oder p-Heptanoyloxyphenyl, o-, m- oder p-Octanoyloxyphenyl, o-, m- oder p-Nonanoyloxyphenyl, o-, m- oder p-Decanoyloxyphenyl; weiterhin Dihalogenphenyl wie 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2-Fluor-4-chlorphenyl, 2-Brom-4-chlorphenyl; Dimethylphenyl wie 2,3-, 2,4-, 2,5-, 2,6-, 3,4- oder 3,5-Dimethylphenyl; Dimethoxyphenyl wie 2,3-, 2,4-, 2,5-, 2,6-, 3,4- oder 3,5-Dimethoxyphenyl; Hydroxychlor-phenyl wie 2-Hydroxy-4-chlorphenyl, 3-Hydroxy-4-chlor-phenyl; Alkanoyloxy-chlor-phenyl, insbesondere 2-Alkanoyloxy-4-chlor-phenyl wie 2-Acetoxy-, 2-Propionyloxy-, 2-Butyryloxy-, 2-Valeryloxy-, 2-Hexanoyloxy-, 2-Heptanoyloxy, 2-Octanoyloxy-, 2-Nonanoyloxy- oder 2-Decanoyloxy-4-chlor-phenyl; Alkoxy-trifluormethyl-phenyl, insbesondere Methoxy-trifluormethyl-phenyl wie 2-Methoxy-5-trifluormethylphenyl.

Der Rest $R^1$ bedeutet vorzugsweise H oder Methyl, ferner Äthyl, n-Propyl, Allyl, Cyclopropylmethyl oder Benzyl. Der Rest $R^2$ bedeutet vorzugsweise H, ferner Methyl oder Phenyl.

Dementsprechend bedeutet der Rest R vorzugsweise 2-(2-Pyrrolidyl)-äthyl, 2-(1-Methyl-2-pyrrolidyl)-äthyl, 2-(2-Piperidyl)-äthyl, 2-(1-Methyl-2-piperidyl)-äthyl, ferner z.B. 2-(1-Äthyl-, 2-(1-n-Propyl-, 2-(1-Allyl-, 2-(1-Cyclopropylmethyl- oder 2-(1-Benzyl-2-pyrrolidyl)-äthyl, 2-(1-Äthyl-, 2-(1-n-Propyl-, 2-(1-Allyl-, 2-(1-Cyclopropylmethyl- oder 2-(1-Benzyl-2-piperidyl)-äthyl, 1-Methyl-2-(2-pyrrolidyl)-äthyl, 1-Phenyl-2-(2-pyrrolidyl)-äthyl, 1-Methyl-2-(1-methyl-2-pyrrolidyl)-äthyl, 1-Phenyl-2-(1-methyl-2-pyrrolidyl)-äthyl, 1-Methyl-2-(2-piperidyl)-äthyl, 1-Phenyl-2-(2-piperidyl)-äthyl, 1-Methyl-2-(1-methyl-2-piperidyl)-äthyl, 1-Phenyl-2-(1-methyl-2-piperidyl)-äthyl, 3-Methyl- oder 1,3-Dimethyl-4-hexahydroazepinyl.

Dementsprechend sind Gegenstand der Erfindung insbesondere diejenigen Verbindungen der Formel I, in denen mindestens einer der genannten Reste eine der vorstehend angegebenen,

insbesondere der vorstehend angegebenen bevorzugten Bedeutungen hat. Einige bevorzugte Gruppen von Verbindungen können durch die folgenden TeilformelnIa bis Ie ausgedrückt werden, die der Formel I entsprechen und worin die nicht näher bezeichneten Reste die bei Formel I angegebene Bedeutung haben, worin jedoch

in Ia Ar Phenyl, Fluorphenyl, Chlorphenyl, Bromphenyl, Tolyl, Methoxyphenyl, Cyclopentyloxyphenyl, Trifluormethylphenyl, Methylthiophenyl oder Hydroxyphenyl bedeutet;

in Ib Ar Phenyl, p-Fluorphenyl, o- oder p-Chlorphenyl, o- oder p-Bromphenyl, p-Tolyl, o- oder p-Methoxyphenyl, o-Cyclopentyloxyphenyl, m-Trifluormethylphenyl, p-Methylthiophenyl oder p-Hydroxyphenyl bedeutet;

in Ic R 2-(1-Methyl-2-pyrrolidyl)-äthyl, 2-(2-Piperidyl)-äthyl, 2-(1-Methyl-2-piperidyl)-äthyl, 1-Phenyl-2-(1-methyl-2-piperidyl)-äthyl oder 1,3-Dimethyl-4-hexahydroazepinyl bedeutet;

in Id Ar Phenyl, p-Fluorphenyl, o- oder p-Chlorphenyl, o- oder p-Bromphenyl, p-Tolyl, o- oder p-Methoxyphenyl, o-Cyclopentyloxyphenyl, m-Trifluormethylphenyl, p-Methylthiophenyl oder p-Hydroxyphenyl und

R 2-(1-Methyl-2-pyrrolidyl)-äthyl, 2-(2-Piperidyl)-äthyl, 2-(1-Methyl-2-piperidyl)-äthyl, 1-Phenyl-2-(1-methyl-2-piperidyl)-äthyl oder 1,3-Dimethyl-4-hexahydroazepinyl bedeutet;

 

in Ie Ar p-Fluorphenyl, p-Chlorphenyl, p-Methoxyphenyl, m-Trifluormethylphenyl oder p-Methylthiophenyl und

R 2-(1-Methyl-2-pyrrolidyl)-äthyl, 2-(2-Piperidyl)-äthyl, 2-(1-Methyl-2-piperidyl)-äthyl oder 1,3-Dimethyl-4-hexahydroazepinyl bedeutet.

Die Verbindungen der Formel I besitzen mindestens ein asymmetrisches Kohlenstoffatom. Sie können daher als Racemate, falls mehrere asymmetrische Kohlenstoffatome vorhanden sind, auch als Gemische mehrererRacemate wowie in verschiedenen optisch-aktiven Formen vorliegen.

Gegenstand der Erfindung ist ferner ein Verfahren zur Herstellung der Verbindungen der Formel I sowie ihrer physiologisch unbedenklichen Säureadditionssalze, dadurch gekennzeichnet, daß man ein Phenol der allgemeinen Formel II

Ar-OH II

worin

Ar die angegebene Bedeutung hat,

oder eines seiner Salze mit einer Verbindung der allgemeinen Formel III

X-R III

worin

X Cl, Br, J oder OH bedeutet und

R die angegebene Bedeutung hat

oder mit einem ihrer reaktionsfähigen Derivate umsetzt oder daß man eine Verbindung der allgemeinen Formel IV

$Ar^1$-O-$R^3$ IV

worin

$Ar^1$ Ar oder einen zu Ar reduzierbaren Rest und

$R^3$ R oder einen zu R reduzierbaren Rest bedeuten und

Ar und R die angegebenen Bedeutungen haben, worin jedoch nicht gleichzeitig $Ar^1$ = Ar und $R^3$ = R sein dürfen,

mit einem Reduktionsmittel behandelt und daß man gegebenenfalls in einer erhaltenen Verbindung der Formel I eine sekundäre Aminogruppe durch Behandeln mit einem alkylierenden, alkenylierenden, cyclopropylmethylierenden oder benzylierenden Mittel in die entsprechende tertiäre Aminogruppe oder eine N-Benzylgruppe durch Behandeln mit einem Reduktionsmittel in eine NH-Gruppe und/oder eine phenolische Hydroxygruppe durch Behandeln mit einem alkylierenden, cycloalkylierenden oder alkanoylierenden Mittel in die entsprechende Alkoxy-, Cycloalkoxy- oder Alkanoyloxygruppe umwandelt und/oder eine erhaltene Base der Formel I durch Behandeln mit einer Säure in eines ihrer physiologisch unbedenklichen Säureadditionssalze umwandelt.

Die Herstellung der Verbindungen der Formel I erfolgt im übrigen nach an sich bekannten Methoden, wie sie in der Literatur (z.B. in den Standardwerken wie Houben-Weyl, Methoden der Organischen Chemie, Georg-Thieme-Verlag, Stuttgart; Organic Reactions, John Wiley & Sons, Inc., New York) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe der Formeln II bis IV können gewünschtenfalls auch in situ gebildet werden, derart, daß man sie aus dem Reaktionsgemisch nicht isoliert, sondern sofort weiter zu den Verbindungen der Formel I umsetzt.

Die Verbindungen der Formel I werden vorzugsweise durch Reaktion der Phenole der Formel Ar-OH (II) oder - bevorzugt - ihrer Salze mit den Verbindungen der Formel X-R (III) erhalten.

Die Phenole der Formel II sind in der Regel bekannt. Falls sie neu sind, können sie nach an sich bekannten Methoden hergestellt werden, z. B. durch Spaltung entsprechender Benzyl- oder Methyläther.

In den Basen der Formel III bedeutet der Rest X vorzugsweise Cl oder Br. Reaktionsfähige Derivate der Basen der Formel III sind insbesondere die reaktionsfähigen Ester der Alkohole der Formel III (X = OH), vorzugsweise die entsprechenden Alkylsulfonate (worin die Alkylgruppe 1 - 6 C-Atome besitzt) und die entsprechenden Arylsulfonate (worin die Arylgruppe 6 - 10 C-Atome besitzt), z.B. die entsprechenden Methan-, Benzol-, p-Toluol- oder Naphthalin-1- oder -2-sulfonate.

Die Basen der Formel III sind teilweise bekannt. Die bisher noch nicht bekannten Basen der Formel III können nach an sich bekannten Methoden in Analogie zu bekannten Verbindungen hergestellt werden. So können die Verbindungen der Formel III (X = OH) z.B. durch Reduktion entsprechender Ester oder Ketone vom Typ (1-$R^1$-2-pyrrolidyl)-$CH_2$-COO-Alkyl, (1-$R^1$-2-pyrrolidyl)-$CH_2$-CO-$R^2$, (1-$R^1$-2-piperidyl)-$CH_2$-COOAlkyl, (1-$R^1$-2-piperidyl)-$CH_2$-CO-$R^2$ oder 1-$R^1$-3-Z-hexahydroazepin-4-on erhältlich, die Verbindungen der Formel III (X = Cl, Br oder J) aus den Alkoholen mit anorganischen Halogeniden wie $SOCl_2$, $PBr_3$ oder HJ, die Sulfonate durch Veresterung der Alkohole mit den entsprechenden Sulfonylchloriden. Die tertiären unter den Aminen der Formel II (in denen $R^1$ nicht H bedeutet) sind auch aus den sekundären Aminen (III, $R^1$ = H)

durch Alkylierung, Alkenylierung, Cyclopropylmethylierung oder Benzylierung zugänglich. Umgekehrt können die sekundären Amine (III, $R^1$ = H) aus den entsprechenden N-Alkylderivaten (III, $R^1$ = Alkyl mit 1 - 4 C-Atomen) durch Desalkylierung mit Chlorameisensäureäthylester erhalten werden. Weiterhin können die Amine der Formel III durch Reduktion entsprechender Pyridine, Pyrrole oder Azepine hergestellt werden. So kann man z. B. 2-Methylpyridin mit $C_6H_5Li$ metallieren, anschließend mit Benzaldehyd zu 1-Phenyl-2-(2-pyridyl)-äthanol umsetzen und dieses zu 1-Phenyl-2-(2-piperidyl)-äthanol reduzieren. In ähnlicher Weise kann man 1-Phenyl-2-(1-methyl-2-pyrrolidyl)-äthanol erhalten durch Reaktion von 2-Hydroxymethylpyrrolidin mit $SOCl_2$ und anschließende Leuckart-Wallach-Methylierung zu 1-Methyl-2-chlormethylpyrrolidin, Überführung in das Grignard-Derivat und Reaktion mit Benzaldehyd. 1,3-Dimethyl-4-hydroxy-hexahydroazepin ist zugänglich durch Ringerweiterung von 1,3-Dimethyl-4-piperidon mit $CH_2N_2$ zu 1,3-Dimethyl-hexahydroazepin-4-on und Reduktion mit $NaBH_4$.

Vor der Umsetzung mit III wird das Phenol II zweckmäßig zunächst in ein Salz übergeführt, insbesondere in ein Metallsalz, z. B. ein Alkalimetallsalz (Li-, Na- oder K-Salz). Man kann das Phenol mit einem metallsalz-bildenden Reagenz umsetzen, z. B. einem Alkalimetall (z. B. Na), einem Alkalimetallhydrid oder -amid (z. B. LiH, NaH, $NaNH_2$ oder $KNH_2$), einem Alkalimetallalkoholat (worin der Alkohol-Teil vorzugsweise 1 - 4 C-Atome besitzt, z. B. Lithium-, Natrium- oder Kaliummethylat, -äthylat oder -tert.-butylat), einer Organometallverbindung (z. B. Butyllithium, Phenyllithium oder Phenylnatrium), einem Metallhydroxid, -carbonat oder -bicarbonat (z. B. des Li, Na, K oder Ca). Die Herstellung des Phenolats wird vorteilhafterweise in Gegenwart eines Lösungsmittels oder Lösungsmittelgemisches vorgenommen. Geeignete Lösungsmittel sind z. B. Kohlenwasserstoffe (wie Hexan, Benzol, Toluol oder Xylol), halogenierte Kohlenwasserstoffe

(wie $CH_2Cl_2$, $CHCl_3$ oder $CCl_4$), Aether (wie Diäthyläther, Diisopropyläther, Tetrahydrofuran (THF), Dioxan oder Diäthylenglykoldimethyläther), Amide [wie Dimethylformamid (DMF)], Alkohole (wie Methanol oder Aethanol), Ketone (wie Aceton oder Butanon).

Das Phenol II oder dessen Salz wird mit der Verbindung III vorzugsweise in Gegenwart eines Verdünnungsmittel umgesetzt, z. B. desjenigen Lösungsmittels, das für die Herstellung des Salzes verwendet worden ist, das jedoch durch ein anderes Lösungsmittel ersetzt oder mit einem solchen verdünnt sein kann. Eine besondere Variante besteht darin, zweiphasig (z. B. in $CH_2Cl_2$/wässeriger Natronlauge) zu arbeiten, wobei auch ein Katalysator (z. B. ein Kronenäther, ein Ammoniumsalz wie ein Trialkylbenzylammoniumhalogenid oder ein Phosphoniumsalz) zugesetzt werden kann. Die Umsetzung wird in der Regel bei Temperaturen zwischen etwa -20 und 150° durchgeführt, vorzugsweise zwischen 20 und 120°.

Das Phenolat kann auch in situ gebildet werden. In diesem Falle läßt man das Phenol II und die Verbindung III miteinander in Gegenwart einer Base reagieren.

Die Verbindungen der Formel I sind weiterhin durch Reduktion der Verbindungen der Formel IV erhältlich. In diesem ist mindestens einer der beiden Reste $Ar^1$ bzw. $R^3$ zu Ar bzw. zu R reduzierbar, so daß die Formel IV die allgemeinen Teilformeln IVa, IVb und IVc umfaßt

| $Ar-O-R^4$ | $Ar^2-O-R$ | $Ar^2-O-R^4$ |
|---|---|---|
| IVa | IVb | IVc |

worin

$Ar^2$ einen zu Ar reduzierbaren Rest und
$R^4$ einen zu R reduzierbaren Rest bedeutet und
Ar und R die angegebenen Bedeutungen haben.

Der Rest $Ar^2$ ist bevorzugt eine ein- oder mehrfach durch Benzyloxy substituierte Phenylgruppe, die auch einen oder mehrere weitere Substituenten enthalten kann, insbesondere o-, m- oder p-Benzyloxyphenyl oder 2-Benzyloxy-4-chlorphenyl.

Der Rest $R^4$ unterscheidet sich vom Rest R durch (vorzugsweise 1 - 4) zusätzliche C-C- und/oder C-N-Mehrfachbindungen und/oder das Vorhandensein einer oder mehrerer (vorzugsweise 1 - 3) reduzierbarer Gruppe(n), insbesondere Carbonyl- an Stelle von $CH_2$-Gruppen. Bevorzugte Reste $R^4$ sind z.B. (2-Pyridyl)-$CH_2$-$CHR^2$-, (1-$R^1$-3-, -4-, -5- oder -6-oxo-2-piperidyl)-$CH_2$-$CHR^2$, ferner (1-$R^1$-2-pyrryl)-$CH_2$-$CHR^2$-, (1-$R^1$-3-, -4- oder -5-oxo-2-pyrrolidyl)-$CH_2$-$CHR^2$-, (1-$R^1$-2-pyrrolidyl)-CO-$CHR^2$-, (1-$R^1$-2-pyrrolidyl)-$CH_2$-CO-, (1-$R^1$-2-piperidyl)-CO-$CHR^2$-, (1-$R^1$-2-piperidyl)-$CH_2$-CO-, 1-$R^1$-3-Z-4-azepinyl, 1-$R^1$-3-Z-2-, -5-, -6- oder -7-oxo-hexahydroazepinyl.

Die Ausgangsstoffe der Formel IV sind in der Regel neu. Sie sind jedoch leicht in Analogie zu bekannten Verbindungen nach an sich bekannten Methoden erhältlich, vorzugsweise durch Reaktion der meist bekannten Phenole der allgemeinen Formel $Ar^1$-OH oder ihrer Salze mit Verbindungen der allgemeinen Formel X-$R^3$ (worin $Ar^1$, X und $R^3$ die oben angegebenen Bedeutungen haben). Die Verbindungen der Formel X-$R^3$ sind in Analogie zu den Verbindungen der Formel III herstellbar.

Die Ausgangsstoffe der Formel IV können beispielsweise durch katalytische Hydrierung, mit nascierendem Wasserstoff, mit komplexen Metallhydriden oder mit Hilfe anderer chemischer Reduktionsmittel in die Verbindungen der Formel I umgewandelt werden. Die für die einzelnen Ausgangsstoffe geeignetsten Reduktionsmethoden sind im allgemeinen von der Art der reduzierbaren Gruppe $Ar^2$ bzw. $R^4$ abhängig und dem Fachmann nach den Angaben der Literatur geläufig. So können z. B. Pyridinderivate und Benzyläther besonders vorteilhaft katalytisch hydriert werden. Eine Reduktion von Lactamen erfolgt dagegen besonders vorteilhaft mit komplexen Metallhydriden oder mit Diboran.

Für katalytische Hydrierungen eignen sich beispielsweise Edelmetall-, Nickel- oder Kobaltkatalysatoren, ferner auch Mischkatalysatoren wie Kupferchromoxid. Als Edelmetalle kommen in erster Linie Platin und Palladium in Betracht, die auf Trägern (z. B. auf Kohle, Calciumcarbonat oder Strontiumcarbonat), als Oxide (z. B. Platinoxid) oder in feinteiliger Form vorliegen können. Nickel- und Kobaltkatalysatoren werden zweckmäßig als Raney-Metalle eingesetzt. Man kann zweckmäßig bei Drucken zwischen etwa 1 und 200 at und bei Temperaturen zwischen etwa -80 und $+150^o$, vorzugsweise zwischen 20 und $100^o$ hydrieren. Die Hydrierung erfolgt in Gegenwart eines inerten Lösungsmittels, z. B. eines Alkohols wie Methanol, Äthanol oder Isopropanol, einer Carbonsäure wie Essigsäure, eines Esters wie Äthylacetat, eines Äthers wie THF oder Dioxan. Man kann auch Lösungsmittelgemische verwenden, z. B. auch Wasser enthaltende Gemische. Weiterhin kann es vorteilhaft sein, eine Base wie Natrium- oder Kaliumhydroxid oder Ammoniak bei der Hydrierung zuzufügen.

Ferner können als Reduktionsmittel komplexe Metallhydride wie $LiAlH_4$, $NaBH_4$ oder $NaAl(OCH_2CH_2OCH_3)_2H_2$ sowie Diboran eingesetzt werden, falls erwünscht unter Zusatz von Katalysatoren wie $BF_3$, $AlCl_3$ oder LiBr. Als Lösungsmittel eignen sich hierfür insbesondere Äther wie Diäthyläther, THF, Dioxan, 1,2-Dimethoxyäthan oder Diglyme sowie Kohlenwasserstoffe wie Benzol. Für eine Reduktion mit $NaBH_4$ sind in erster Linie Alkohole wie Methanol oder Äthanol als Lösungsmittel geeignet. Nach dieser Methode reduziert man vorzugsweise bei Temperaturen zwischen etwa -80 und +150°, insbesondere zwischen etwa 20 und 120°.

Weiterhin ist als Reduktionsmethode die Umsetzung mit nascierendem Wasserstoff geeignet. Dieser kann beispielsweise durch Behandlung von Metallen mit Säuren oder Basen erzeugt werden. So können z. B. die Systeme Zink/Säure, Zink/Alkalilauge, Eisen/Säure oder Zinn/Säure verwendet werden. Als Säuren eignen sich z. B. Salzsäure oder Essigsäure. Auch ein Alkalimetall wie Natrium in einem Alkohol wie Äthanol, Isopropanol, n-Butanol, Amylalkohol, Isoamylalkohol oder in Phenol kann als Reduktionsmittel verwendet werden, ferner z. B. eine Aluminium-Nickel-Legierung in alkalisch-wässeriger oder alkalisch-wässerig-alkoholischer Lösung, sowie Natrium- oder Aluminiumamalgam in wässerig-alkoholischer oder wässeriger Lösung. Bei diesen Methoden liegen die Reaktionstemperaturen zwischen etwa 0 und etwa 150°, vorzugsweise zwischen etwa 20 und 120°.

Die Ausgangsverbindungen der Formel IV können auch durch kathodische Reduktion in Verbindungen der Formel I umgewandelt werden, zweckmäßig in wässerig-alkoholischem oder wässerig-essigsaurem Medium. Weitere geeignete Reduktionsmittel sind beispielsweise Natriumdithionit in wässerig-alkoholischer oder alkalischer Lösung, ferner Eisen(II)-hydroxid, Zinn(II)-chlorid, Schwefelwasserstoff, Hydrogensulfide, Sulfide, Polysulfide, Hydrazin, die sämtlich nach den in der Literatur für derartige Reduktionen angegebenen Bedingungen zur Anwendung kommen.

Weiterhin kann man gewünschtenfalls ein erhaltenes sekundäres Amin der Formel I ($R^1$ = H) am Stickstoffatom alkylieren, wobei man tertiäre Amine der Formel I ($R^1$ = Alkyl mit 1 - 4 C-Atomen) erhält. Als N-Alkylierungsmittel eignen sich z. B. die entsprechenden Alkylhalogenide, z. B. Methylchlorid, Methylbromid, Methyljodid, Äthylchlorid, Äthylbromid, Äthyljodid, n-Propylchlorid, -bromid oder -jodid usw., ferner die entsprechenden Dialkylsulfate wie Dimethylsulfat und die entsprechenden Sulfonsäurealkylester wie p-Toluolsulfonsäure-methylester. Eine Methylgruppe kann ferner beispielsweise durch Behandeln mit Ameisensäure und wässeriger Formaldehydlösung eingeführt werden, zweckmäßig durch mehrstündiges Erhitzen auf Temperaturen zwischen 50 und 100°. Allgemein wird die N-Alkylierung zweckmäßig in Gegenwart oder Abwesenheit eines inerten Lösungsmittels bei Temperaturen zwischen etwa 0 und etwa 120°, vorzugsweise zwischen 40 und 100° vorgenommen, wobei auch ein Katalysator zugegen sein kann, vorzugsweise eine Base wie Kalium-tert.-butylat.

Eine Alkylierung gelingt auch durch Behandeln einer sekundären Base I ($R^1$ = H) mit einem Aldehyd oder Keton in Gegenwart von Wasserstoff und einem Hydrierungskatalysator (z. B. Raney-Nickel) bei Temperaturen zwischen etwa 50 und 100° und Drucken zwischen etwa 1 und 200 at; so erhält man mit Aceton die entsprechende Isopropylverbindung I ($R^1$ = Isopropyl).

Eine Alkylierung ist auch mehrstufig möglich. Man kann beispielsweise eine Verbindung der Formel I ($R^1$ = H) zunächst in an sich bekannter Weise acylieren (z. B. durch Behandln mit Acetanhydrid/Pyridin acetylieren) und das erhaltene N-Acylierungsprodukt (z. B. N-Acetylierungsprodukt) anschließend zum gewünschten tertiären Amin reduzieren, beispielsweise mit einem komplexen Metallhydrid wie $LiAlH_4$ in einem inerten Lösungsmittel wie Diäthyläther oder THF, vorzugsweise bei Temperaturen zwischen 20 und 60°.

In ganz analoger Weise kann ein sekundäres Amin der Formel I ($R^1$ = H) mit alkenylierdenen, cyclopropylmethylierenden oder benzylierenden Mitteln (z. B. Alkenylhalogeniden wie Allylchlorid oder -bromid, Cyclopropylmethylhalogeniden wie Cyclopropylmethylchlorid oder -bromid, Benzylhalogeniden wie Benzylchlorid oder -bromid) behandelt werden, wobei Verbindungen der Formel I ($R^1$ = Alkenyl mit bis zu 4 C-Atomen, Cyclopropylmethyl oder Benzyl) gebildet werden.

Weiterhin kann in einer Verbindung der Formel I ($R^1$ = Benzyl) die Benzylgruppe nach einer der oben angegebenen Methoden reduktiv entfernt werden, vorzugsweise durch Hydrogenolyse in Gegenwart eines Edelmetallkatalysators.

Ferner können in einer erhaltenen Verbindung der Formel I phenolische Hydroxygruppen alkyliert, cycloalkyliert oder acyliert werden. Als Alkylierungsmittel eignen sich z.B. die oben angegebenen Alkylhalogenide, Dialkylsulfate und Sulfonsäurealkylester, als Cycloalkylierungsmittel die entsprechenden Cycloalkylhalogenide (z. B. Cyclopropylchlorid, -bromid oder -jodid, Cyclobutylchlorid, -bromid oder -jodid, Cyclopentylchlorid, -bromid oder -jodid, Cyclohexylchlorid, -bromid oder -jodid), als Acylierungsmittel die entsprechenden Fettsäuren (z. B. Ameisen-,

Essig-, Propion-, Butter-, Valerian-, Capron-, Hexan-, Heptan-, Octan-, Nonan- oder Decansäure) sowie ihre Halogenide, insbesondere ihre Chloride und Bromide, und ihre Anhydride. Diese Umsetzungen werden zweckmäßig bei Temperaturen zwischen etwa 0 und etwa 150°, vorzugsweise zwischen 20 und 100°, in einem inerten Lösungsmittel vorgenommen, z. B. einem Alkohol wie Methanol oder Äthanol, einem Kohlenwasserstoff wie Benzol, einem Amid wie DMF, einem Äther wie THF oder einem Amin wie Pyridin. Verwendet man Halogenide oder Anhydride, so arbeitet man zweckmäßig in Gegenwart einer Base wie NaOH, KOH, Triäthylamin oder Pyridin, wobei ein Überschuß dieser Base als Lösungsmittel dienen kann. Acylierungen mit den freien Fettsäuren gelingen zweckmäßig in Gegenwart eines wasserabspaltenden Mittels wie Carbonyldiimidazol oder Dicyclohexylcarbodiimid.

Eine erhaltene Base der Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z. B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diäthylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Äthansulfonsäure, Äthandisulfonsäure, 2-Hydroxyäthansulfonsäure, Benzolsulfonsäure, p-Toluolsulfonsäure, Naphthalin-mono- und -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden.

Gegenstand der Erfindung ist ferner die Verwendung der Verbindungen der Formel I und ihrer physiologisch unbedenklichen Salze zur Herstellung pharmazeutischer Zubereitungen, insbesondere auf nicht-chemischem Wege. Hierbei können sie zusammen mit mindestens einem Träger- oder Hilfsstoff und gegebenenfalls in Kombination mit einem oder mehreren weiteren Wirkstoff(en) in eine geeignete Dosierungsform gebracht werden.

Gegenstand der Erfindung sind ferner Mittel, insbesondere pharmazeutische Zubereitungen, enthaltend eine Verbindung der Formel I und/oder eines ihrer physiologisch unbedenklichen Säureadditionssalze. Diese Zubereitungen können als Arzneimittel in der Human- oder Veterinärmedizin eingesetzt werden. Als Trägersubstanzen kommen organische oder anorganische Stoffe in Frage, die sich für die enterale (z. B. orale), parenterale oder topikale Applikation eignen und mit den neuen Verbindungen nicht reagieren, beispielsweise Wasser, pflanzliche Öle, Benzylalkohole, Polyäthylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositorien, zur parenteralen Applikation Lösungen, vorzugsweise ölige oder wässerige Lösungen, ferner Suspensionen, Emulsionen oder Implantate, für die topikale Anwendung Salben, Cremes oder Puder. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z. B. ein oder mehrere Vitamine.

Gegenstand der Erfindung ist ferner die Verwendung der Verbindungen der Formel I und ihrer physiologisch unbedenklichen Säureadditionssalze bei der Bekämpfung von Krankheiten, insbesondere von Depressionen verschiedener Ätiologie und Symptomatologie, sowie ihre Verwendung bei der therapeutischen Behandlung des menschlichen oder tierischen Körpers.

Dabei werden die erfindungsgemäßen Substanzen in der Regel in Analogie zu bekannten, im Handel befindlichen Psychopharmaka (z. B. Imipramin) verabreicht, vorzugsweise in Dosierungen zwischen etwa 2 und 500 mg, insbesondere zwischen 10 und 50 mg pro Dosierungseinheit. Die tägliche Dosierung liegt vorzugsweise zwischen etwa 0,05 und 10mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Jede der in den folgenden Beispielen genannten Verbindungen der Formel I ist zur Herstellung von pharmazeutischen Zubereitungen besonders geeignet.

In den nachfolgenden Beispielen bedeutet "übliche Aufarbeitung":

Man gibt, falls erforderlich, Wasser hinzu, extrahiert mit einem organischen Lösungsmittel wie Benzol, Chloroform oder Dichlormethan, trennt ab, trocknet die organische Phase über Natriumsulfat, filtriert, dampft ein und reinigt durch Chromatographie und/oder Kristallisation.

Die Rf-Werte wurden an Kieselgel mit Toluol/Triäthylamin (8:2) erhalten, wenn nicht anders angegeben.

Beispiel 1

Man löst 5,1 g p-Chlorphenol in 20 ml absolutem Äthanol, gibt 60 ml 0,5 n äthanolisches KOH zu und rührt eine Stunde bei 20°. Es wird eingedampft und der Rückstand in 100 ml absolutem DMF aufgenommen. Man gibt 1,62 g 1-Methyl-2-(2-chloräthyl)-piperidin hinzu, kocht das Gemisch 4 Stunden, dampft erneut ein, arbeitet wie üblich auf und erhält 1-Methyl-2-(2-p-chlorphenoxyäthyl)-piperidin. Hydrochlorid, F. 139-140°.

Beispiele 2 bis 451

Analog Beispiel 1 erhält man aus den entsprechenden Phenolen der Formel II mit 2-(2-Chloräthyl)-pyrrolidin, 1-Methyl-2-(2-chloräthyl)-pyrrolidin, 2-(2-Chlor-2-phenyläthyl)-pyrrolidin, 1-Methyl-2-(2-chlor-2-phenyläthyl)-pyrrolidin, 2-(2-Chloräthyl)-piperidin, 1-Methyl-2-(2-chloräthyl)-piperidin, 2-(2-Chlor-2-phenyläthyl)-piperidin, 1-Methyl-2-(2-chlor-2-phenyläthyl)-piperidin, 3-Methyl-4-chlor-hexahydroazepin, 1,3-Dimethyl-4-chlorhexahydroazepin oder den entsprechenden Bromverbindungen:

2. 2-(2-Phenoxyäthyl)-pyrrolidin.
3. 2-(2-o-Fluorphenoxyäthyl)-pyrrolidin.
4. 2-(2-m-Fluorphenoxyäthyl)-pyrrolidin.
5. 2-(2-p-Fluorphenoxyäthyl)-pyrrolidin.
6. 2-(2-o-Chlorphenoxyäthyl)-pyrrolidin.
7. 2-(2-m-Chlorphenoxyäthyl)-pyrrolidin.
8. 2-(2-p-Chlorphenoxyäthyl)-pyrrolidin.
9. 2-(2-o-Bromphenoxyäthyl)-pyrrolidin.
10. 2-(2-m-Bromphenoxyäthyl)-pyrrolidin.
11. 2-(2-p-Bromphenoxyäthyl)-pyrrolidin.
12. 2-(2-o-Tolyloxyäthyl)-pyrrolidin.
13. 2-(2-m-Tolyloxyäthyl)-pyrrolidin.
14. 2-(2-p-Tolyloxyäthyl)-pyrrolidin.
15. 2-(2-o-Methoxyphenoxyäthyl)-pyrrolidin.
16. 2-(2-m-Methoxyphenoxyäthyl)-pyrrolidin.
17. 2-(2-p-Methoxyphenoxyäthyl)-pyrrolidin.
18. 2-(2-o-Cyclopentyloxyphenoxyäthyl)-pyrrolidin.
19. 2-(2-m-Cyclopentyloxyphenoxyäthyl)-pyrrolidin.
20. 2-(2-p-Cyclopentyloxyphenoxyäthyl)-pyrrolidin.
21. 2-(2-o-Cyclohexyloxyphenoxyäthyl)-pyrrolidin.
22. 2-(2-m-Cyclohexyloxyphenoxyäthyl)-pyrrolidin.
23. 2-(2-p-Cyclohexyloxyphenoxyäthyl)-pyrrolidin.
24. 2-(2-o-Trifluormethylphenoxyäthyl)-pyrrolidin.
25. 2-(2-m-Trifluormethylphenoxyäthyl)-pyrrolidin.
26. 2-(2-p-Trifluormethylphenoxyäthyl)-pyrrolidin.
27. 2-(2-o-Cyanphenoxyäthyl)-pyrrolidin.
28. 2-(2-m-Cyanphenoxyäthyl)-pyrrolidin.
29. 2-(2-p-Cyanphenoxyäthyl)-pyrrolidin.
3o! 2-(2-o-Methylthiophenoxyäthyl)-pyrrolidin.
31. 2-(2-m-Methylthiophenoxyäthyl)-pyrrolidin.
32. 2-(2-p-Methylthiophenoxyäthyl)-pyrrolidin.
33. 2-(2-o-Trifluormethylthiophenoxyäthyl)-pyrrolidin.
34. 2-(2-m-Trifluormethylthiophenoxyäthyl)-pyrrolidin.
35. 2-(2-p-Trifluormethylthiophenoxyäthyl)-pyrrolidin.
36. 2-(2-o-Hydroxyphenoxyäthyl)-pyrrolidin.
37. 2-(2-m-Hydroxyphenoxyäthyl)-pyrrolidin.

38. 2-(2-p-Hydroxyphenoxyäthyl)-pyrrolidin.
39. 2-(2-o-Acetoxyphenoxyäthyl)-pyrrolidin.
40. 2-(2-m-Acetoxyphenoxyäthyl)-pyrrolidin.
41. 2-(2-p-Acetoxyphenoxyäthyl)-pyrrolidin.
42. 2-(2-p-Propionyloxyphenoxyäthyl)-pyrrolidin.
43. 2-(2-p-Butyryloxyphenoxyäthyl)-pyrrolidin.
44. 2-(2-p-Valeryloxyphenoxyäthyl)-pyrrolidin.
45. 2-(2-p-Hexanoyloxyphenoxyäthyl)-pyrrolidin.
46. 2-(2-p-Heptanoyloxyphenoxyäthyl)-pyrrolidin.
47. 2-/2-(3,4-Dimethoxyphenoxy)-äthyl/-pyrrolidin.
48. 2-/2-(2-Methoxy-5-trifluormethylphenoxy)-äthyl/-pyrrolidin.
49. 2-/2-(2-Hydroxy-4-chlorphenoxy)-äthyl/-pyrrolidin.
50. 2-/2-(2-Heptanoyloxy-4-chlorphenoxy)-äthyl/-pyrrolidin.
51. 2-/2-(2-Decanoyloxy-4-chlorphenoxy)-äthyl/-pyrrolidin.
52. 1-Methyl-2-(2-phenoxyäthyl)-pyrrolidin.
53. 1-Methyl-2-(2-o-fluorphenoxyäthyl)-pyrrolidin.
54. 1-Methyl-2-(2-m-fluorphenoxyäthyl)-pyrrolidin.
55. 1-Methyl-2-(2-p-fluorphenoxyäthyl)-pyrrolidin, Öl, Rf 0,77 ($CHCl_3$/Triäthylamin 8:2).
56. 1-Methyl-2-(2-o-chlorphenoxyäthyl)-pyrrolidin.
57. 1-Methyl-2-(2-m-chlorphenoxyäthyl)-pyrrolidin.
58. 1-Methyl-2-(2-p-chlorphenoxyäthyl)-pyrrolidin, Hydrobromid, F. 137-140°.
59. 1-Methyl-2-(2-o-bromphenoxyäthyl)-pyrrolidin.
60. 1-Methyl-2-(2-m-bromphenoxyäthyl)-pyrrolidin.
61. 1-Methyl-2-(2-p-bromphenoxyäthyl)-pyrrolidin.
62. 1-Methyl-2-(2-o-tolyloxyäthyl)-pyrrolidin.
63. 1-Methyl-2-(2-m-tolyloxyäthyl)-pyrrolidin.
64. 1-Methyl-2-(2-p-tolyloxyäthyl)-pyrrolidin.
65. 1-Methyl-2-(2-p-äthylphenoxyäthyl)-pyrrolidin.
66. 1-Methyl-2-(2-p-n-butylphenoxyäthyl)-pyrrolidin.
67. 1-Methyl-2-(2-o-methoxyphenoxyäthyl)-pyrrolidin, Kp. 106-108,5°/0,09 mm.

68. 1-Methyl-2-(2-m-methoxyphenoxyäthyl)-pyrrolidin.
69. 1-Methyl-2-(2-p-methoxyphenoxyäthyl)-pyrrolidin.
70. 1-Methyl-2-(2-p-äthoxyphenoxyäthyl)-pyrrolidin.
71. 1-Methyl-2-(2-p-n-butyloxyphenoxyäthyl)-pyrrolidin.
72. 1-Methyl-2-(2-p-cyclopropyloxyphenoxyäthyl)-pyrrolidin.
73. 1-Methyl-2-(2-o-cyclopentyloxyphenoxyäthyl)-pyrrolidin.
74. 1-Methyl-2-(2-m-cyclopentyloxyphenoxyäthyl)-pyrrolidin.
75. 1-Methyl-2-(2-p-cyclopentyloxyphenoxyäthyl)-pyrrolidin.
76. 1-Methyl-2-(2-o-cyclohexyloxyphenoxyäthyl)-pyrrolidin.
77. 1-Methyl-2-(2-m-cyclohexyloxyphenoxyäthyl)-pyrrolidin.
78. 1-Methyl-2-(2-p-cyclohexyloxyphenoxyäthyl)-pyrrolidin.
79. 1-Methyl-2-(2-o-trifluormethylphenoxyäthyl)-pyrrolidin, Hydrobromid, F. 113-115°.
80. 1-Methyl-2-(2-m-trifluormethylphenoxyäthyl)-pyrrolidin.
81. 1-Methyl-2-(2-p-trifluormethylphenoxyäthyl)-pyrrolidin.
82. 1-Methyl-2-(2-o-cyanphenoxyäthyl)-pyrrolidin.
83. 1-Methyl-2-(2-m-cyanphenoxyäthyl)-pyrrolidin.
84. 1-Methyl-2-(2-p-cyanphenoxyäthyl)-pyrrolidin.
85. 1-Methyl-2-(2-o-methylthiophenoxyäthyl)-pyrrolidin.
86. 1-Methyl-2-(2-m-methylthiophenoxyäthyl)-pyrrolidin.
87. 1-Methyl-2-(2-p-methylthiophenoxyäthyl)-pyrrolidin.
88. 1-Methyl-2-(2-p-äthylthiophenoxyäthyl)-pyrrolidin.
89. 1-Methyl-2-(2-p-n-butylthiophenoxyäthyl)-pyrrolidin.
90. 1-Methyl-2-(2-o-trifluormethylthiophenoxyäthyl)-pyrrolidin.
91. 1-Methyl-2-(2-m-trifluormethylthiophenoxyäthyl)-pyrrolidin.
92. 1-Methyl-2-(2-p-trifluormethylthiophenoxyäthyl)-pyrrolidin.
93. 1-Methyl-2-(2-o-hydroxyphenoxyäthyl)-pyrrolidin.
94. 1-Methyl-2-(2-m-hydroxyphenoxyäthyl)-pyrrolidin.
95. 1-Methyl-2-(2-p-hydroxyphenoxyäthyl)-pyrrolidin.
96. 1-Methyl-2-(2-o-acetoxyphenoxyäthyl)-pyrrolidin.
97. 1-Methyl-2-(2-m-acetoxyphenoxyäthyl)-pyrrolidin.
98. 1-Methyl-2-(2-p-acetoxyphenoxyäthyl)-pyrrolidin

99. 1-Methyl-2-(2-p-propionyloxyphenoxyäthyl)-pyrrolidin.
100. 1-Methyl-2-(2-p-butyryloxyphenoxyäthyl)-pyrrolidin.
101. 1-Methyl-2-(2-p-valeryloxyphenoxyäthyl)-pyrrolidin.
102. 1-Methyl-2-(2-p-hexanoyloxyphenoxyäthyl)-pyrrolidin.
103. 1-Methyl-2-(2-p-heptanoyloxyphenoxyäthyl)-pyrrolidin.
104. 1-Methyl-2-[2-(3,4-dimethoxyphenoxy)-äthyl]-pyrrolidin.
105. 1-Methyl-2-[2-(2-methoxy-5-trifluormethylphenoxy)-äthyl]-pyrrolidin.
106. 1-Methyl-2-[2-(2-hydroxy-4-chlorphenoxy)-äthyl]-pyrrolidin.
107. 1-Methyl-2-[2-(2-heptanoyloxy-4-chlorphenoxy)-äthyl]-pyrrolidin.
108. 1-Methyl-2-[2-(2-decanoyloxy-4-chlorphenoxy)-äthyl]-pyrrolidin.
109. 2-(2-Phenoxy-2-phenyl-äthyl)-pyrrolidin.
110. 2-(2-p-Fluorphenoxy-2-phenyl-äthyl)-pyrrolidin.
111. 2-(2-o-Chlorphenoxy-2-phenyl-äthyl)-pyrrolidin.
112. 2-(2-m-Chlorphenoxy-2-phenyl-äthyl)-pyrrolidin.
113. 2-(2-p-Chlorphenoxy-2-phenyl-äthyl)-pyrrolidin.
114. 2-(2-o-Methoxyphenoxy-2-phenyl-äthyl)-pyrrolidin.
115. 2-(2-p-Methoxyphenoxy-2-phenyl-äthyl)-pyrrolidin.
116. 2-(2-m-Trifluormethylphenoxy-2-phenyl-äthyl)-pyrrolidin.
117. 2-(2-p-Trifluormethylphenoxy-2-phenyl-äthyl)-pyrrolidin.
118. 2-(2-o-Methylthiophenoxy-2-phenyl-äthyl)-pyrrolidin.
119. 2-(2-p-Methylthiophenoxy-2-phenyl-äthyl)-pyrrolidin.
120. 2-(2-p-Trifluormethylthiophenoxy-2-phenyl-äthyl)-pyrrolidin.
121. 1-Methyl-2-(2-phenoxy-2-phenyl-äthyl)-pyrrolidin.
122. 1-Methyl-2-(2-p-fluorphenoxy-2-phenyl-äthyl)-pyrrolidin.
123. 1-Methyl-2-(2-o-chlorphenoxy-2-phenyl-äthyl)-pyrrolidin.
124. 1-Methyl-2-(2-m-chlorphenoxy-2-phenyl-äthyl)-pyrrolidin.
125. 1-Methyl-2-(2-p-chlorphenoxy-2-phenyl-äthyl)-pyrrolidin.
126. 1-Methyl-2-(2-o-methoxyphenoxy-2-phenyl-äthyl)-pyrrolidin.

127. 1-Methyl-2-(2-p-methoxyphenoxy-2-phenyl-äthyl)-pyrrolidin.
128. 1-Methyl-2-(2-m-trifluormethylphenoxy-2-phenyl-äthyl)-pyrrolidin.
129. 1-Methyl-2-(2-p-trifluormethylphenoxy-2-phenyl-äthyl)-pyrrolidin.
130. 1-Methyl-2-(2-o-methylthiophenoxy-2-phenyl-äthyl)-pyrrolidin.
131. 1-Methyl-2-(2-p-methylthiophenoxy-2-phenyl-äthyl)-pyrrolidin.
132. 1-Methyl-2-(2-p-trifluormethylthiophenoxy-2-phenyl-äthyl)-pyrrolidin.
133. 2-(2-Phenoxyäthyl)-piperidin.
134. 2-(2-o-Fluorphenoxyäthyl)-piperidin.
135, 2-(2-m-Fluorphenoxyäthyl)-piperidin.
136. 2-(2-p-Fluorphenoxyäthyl)-piperidin, Hydrochlorid, F. 158-160°.
137. 2-(2-o-Chlorphenoxyäthyl)-piperidin.
138. 2-(2-m-Chlorphenoxyäthyl)-piperidin.
139. 2-(2-p-Chlorphenoxyäthyl)-piperidin, Hydrochlorid, F. 220°.
140. 2-(2-o-Bromphenoxyäthyl)-piperidin.
141. 2-(2-m-Bromphenoxyäthyl)-piperidin.
142. 2-(2-p-Bromphenoxyäthyl)-piperidin.
143. 2-(2-o-Tolyloxyäthyl)-piperidin.
144. 2-(2-m-Tolyloxyäthyl)-piperidin.
145. 2-(2-p-Tolyloxyäthyl)-piperidin.
146. 2-(2-o-Methoxyphenoxyäthyl)-piperidin.
147. 2-(2-m-Methoxyphenoxyäthyl)-piperidin.
148. 2-(2-p-Methoxyphenoxyäthyl)-piperidin, Hydrochlorid, F. 138-140°.
149. 2-(2-o-Cyclopentyloxyphenoxyäthyl)-piperidin.
150. 2-(2-m-Cyclopentyloxyphenoxyäthyl)-piperidin.
151. 2-(2-p-Cyclopentyloxyphenoxyäthyl)-piperidin.
152. 2-(2-o-Cyclohexyloxyphenoxyäthyl)-piperidin.

153. 2-(2-m-Cyclohexyloxyphenoxyäthyl)-piperidin.
154. 2-(2-p-Cyclohexyloxyphenoxyäthyl)-piperidin.
155. 2-(2-o-Trifluormethylphenoxyäthyl)-piperidin.
156. 2-(2-m-Trifluormethylphenoxyäthyl)-piperidin, Hydrochlorid, F. 152-153°.
157. 2-(2-p-Trifluormethylphenoxyäthyl)-piperidin.
158. 2-(2-o-Cyanphenoxyäthyl)-piperidin.
159. 2-(2-m-Cyanphenoxyäthyl)-piperidin.
160. 2-(2-p-Cyanphenoxyäthyl)-piperidin.
161. 2-(2-o-Methylthiophenoxyäthyl)-piperidin.
162. 2-(2-m-Methylthiophenoxyäthyl)-piperidin.
163. 2-(2-p-Methylthiophenoxyäthyl)-piperidin, F. 65-67°.
164. 2-(2-o-Trifluormethylthiophenoxyäthyl)-piperidin.
165. 2-(2-m-Trifluormethylthiophenoxyäthyl)-piperidin.
166. 2-(2-p-Trifluormethylthiophenoxyäthyl)-piperidin.
167. 2-(2-o-Hydroxyphenoxyäthyl)-piperidin.
168. 2-(2-m-Hydroxyphenoxyäthyl)-piperidin.
169. 2-(2-p-Hydroxyphenoxyäthyl)-piperidin.
170. 2-(2-o-Acetoxyphenoxyäthyl)-piperidin.
171. 2-(2-m-Acetoxyphenoxyäthyl)-piperidin.
172. 2-(2-p-Acetoxyphenoxyäthyl)-piperidin.
173. 2-(2-p-Propionyloxyphenoxyäthyl)-piperidin.
174. 2-(2-p-Butyryloxyphenoxyäthyl)-piperidin.
175. 2-(2-p-Valeryloxyphenoxyäthyl)-piperidin.
176. 2-(2-p-Hexanoyloxyphenoxyäthyl)-piperidin.
177. 2-(2-p-Heptanoyloxyphenoxyäthyl)-piperidin.
178. 2-[2-(3,4-Dimethoxyphenoxy)-äthyl]-piperidin.
179. 2-[2-(2-Methoxy-5-trifluormethylphenoxy)-äthyl]-piperidin.
180. 2-[2-(2-Hydroxy-4-chlorphenoxy)-äthyl]-piperidin.
181. 2-[2-(2-Heptanoyloxy-4-chlorphenoxy)-äthyl]-piperidin.
182. 2-[2-(2-Decanoyloxy-4-chlorphenoxy)-äthyl]-piperidin.
183. 1-Methyl-2-(2-phenoxyäthyl)-piperidin, Hydrochlorid, F. 122 - 124°.

184. 1-Methyl-2-(2-o-fluorphenoxyäthyl)-piperidin.
185. 1-Methyl-2-(2-m-fluorphenoxyäthyl)-piperidin.
186. 1-Methyl-2-(2-o-chlorphenoxyäthyl)-piperidin, Hydrochlorid, F. 146 - 148°.
187. 1-Methyl-2-(2-m-chlorphenoxyäthyl)-piperidin.
188. 1-Methyl-2-(2-o-bromphenoxyäthyl)-piperidin.
189. 1-Methyl-2-(2-m-bromphenoxyäthyl)-piperidin.
190. 1-Methyl-2-(2-p-bromphenoxyäthyl)-piperidin.
191. 1-Methyl-2-(2-o-tolyloxyäthyl)-piperidin.
192. 1-Methyl-2-(2-m-tolyloxyäthyl)-piperidin.
193. 1-Methyl-2-(2-p-tolyloxyäthyl)-piperidin, Hydrochlorid, F. 138-139°.
194. 1-Methyl-2-(2-p-äthylphenoxyäthyl)-piperidin.
195. 1-Methyl-2-(2-p-n-butylphenoxyäthyl)-piperidin.
196. 1-Methyl-2-(2-o-methoxyphenoxyäthyl)-piperidin.
197. 1-Methyl-2-(2-m-methoxyphenoxyäthyl)-piperidin.
198. 1-Methyl-2-(2-p-methoxyphenoxyäthyl)-piperidin.
199. 1-Methyl-2-(2-p-äthoxyphenoxyäthyl)-piperidin.
200. 1-Methyl-2-(2-p-n-butyloxyphenoxyäthyl)-piperidin.
201. 1-Methyl-2-(2-p-cyclopropyloxyphenoxyäthyl)-piperidin.
202. 1-Methyl-2-(2-o-cyclopentyloxyphenoxyäthyl)-piperidin.
203. 1-Methyl-2-(2-m-cyclopentyloxyphenoxyäthyl)-piperidin.
204. 1-Methyl-2-(2-p-cyclopentyloxyphenoxyäthyl)-piperidin.
205. 1-Methyl-2-(2-o-cyclohexyloxyphenoxyäthyl)-piperidin.
206. 1-Methyl-2-(2-m-cyclohexyloxyphenoxyäthyl)-piperidin.
207. 1-Methyl-2-(2-p-cyclohexyloxyphenoxyäthyl)-piperidin.
208. 1-Methyl-2-(2-o-trifluormethylphenoxyäthyl)-piperidin.
209. 1-Methyl-2-(2-m-trifluormethylphenoxyäthyl)-piperidin, Hydrochlorid-monohydrat, F. 42-43°.
210. 1-Methyl-2-(2-p-trifluormethylphenoxyäthyl)-piperidin, Hydrochlorid, F. 138 - 139°.
211. 1-Methyl-2-(2-o-cyanphenoxyäthyl)-piperidin.

211. 1-Methyl-2-(2-m-cyanphenoxyäthyl)-piperidin.

213. 1-Methyl-2-(2-p-cyanphenoxyäthyl)-piperidin.

214. 1-Methyl-2-(2-o-methylthiophenoxyäthyl)-piperidin.

215. 1-Methyl-2-(2-m-methylthiophenoxyäthyl)-piperidin.

216. 1-Methyl-2-(2-p-methylthiophenoxyäthyl)-piperidin, Hydrochlorid, F. 143-144°.

217. 1-Methyl-2-(2-p-äthylthiophenoxyäthyl)-piperidin.

218. 1-Methyl-2-(2-p-n-butylthiophenoxyäthyl)-piperidin.

219. 1-Methyl-2-(2-o-trifluormethylthiophenoxyäthyl)-piperidin.

220. 1-Methyl-2-(2-m-trifluormethylthiophenoxyäthyl)-piperidin.

221. 1-Methyl-2-(2-p-trifluormethylthiophenoxyäthyl)-piperidin.

222. 1-Methyl-2-(2-o-hydroxyphenoxyäthyl)-piperidin.

223. 1-Methyl-2-(2-m-hydroxyphenoxyäthyl)-piperidin.

224. 1-Methyl-2-(2-p-hydroxyphenoxyäthyl)-piperidin, Hydrochlorid, F. 182°.

225. 1-Methyl-2-(2-o-acetoxyphenoxyäthyl)-piperidin.

226. 1-Methyl-2-(2-m-acetoxyphenoxyäthyl)-piperidin.

227. 1-Methyl-2-(2-p-acetoxyphenoxyäthyl)-piperidin.

228. 1-Methyl-2-(2-p-propionyloxyphenoxyäthyl)-piperidin.

229. 1-Methyl-2-(2-p-butyryloxyphenoxyäthyl)-piperidin.

230. 1-Methyl-2-(2-p-valeryloxyphenoxyäthyl)-piperidin.

231. 1-Methyl-2-(2-p-hexanoyloxyphenoxyäthyl)-piperidin.

232. 1-Methyl-2-(2-p-heptanoyloxyphenoxyäthyl)-piperidin.

233. 1-Methyl-2-[2-(3,4-dimethoxyphenoxy)-äthyl]-piperidin, Hydrochlorid, F. 176-177°.

234. 1-Methyl-2-[2-(2-methoxy-5-trifluormethylphenoxy)-äthyl]-piperidin.

235. 1-Methyl-2-[2-(2-hydroxy-4-chlorphenoxy)-äthyl]-piperidin.

236. 1-Methyl-2-[2-(2-heptanoyloxy-4-chlorphenoxy)-äthyl]-piperidin.

237. 1-Methyl-2-[2-(2-decanoyloxy-4-chlorphenoxy)-äthyl]-piperidin.

238. 2-(2-Phenoxy-2-phenyl-äthyl)-piperidin.
239. 2-(2-o-Fluorphenoxy-2-phenyl-äthyl)-piperidin.
240. 2-(2-m-Fluorphenoxy-2-phenyl-äthyl)-piperidin.
241. 2-(2-p-Fluorphenoxy-2-phenyl-äthyl)-piperidin.
242. 2-(2-o-Chlorphenoxy-2-phenyl-äthyl)-piperidin.
243. 2-(2-m-Chlorphenoxy-2-phenyl-äthyl)-piperidin.
244. 2-(2-p-Chlorphenoxy-2-phenyl-äthyl)-piperidin.
245. 2-(2-o-Bromphenoxy-2-phenyl-äthyl)-piperidin.
246. 2-(2-m-Bromphenoxy-2-phenyl-äthyl)-piperidin.
247. 2-(2-p-Bromphenoxy-2-phenyl-äthyl)-piperidin.
248. 2-(2-o-Tolyloxy-2-phenyl-äthyl)-piperidin.
249. 2-(2-m-Tolyloxy-2-phenyl-äthyl)-piperidin.
250. 2-(2-p-Tolyloxy-2-phenyl-äthyl)-piperidin.
251. 2-(2-o-Methoxyphenoxy-2-phenyl-äthyl)-piperidin.
252. 2-(2-m-Methoxyphenoxy-2-phenyl-äthyl)-piperidin.
253. 2-(2-p-Methoxyphenoxy-2-phenyl-äthyl)-piperidin.
254. 2-(2-o-Cyclopentyloxy-2-phenyl-äthyl)-piperidin.
255. 2-(2-m-Cyclopentyloxy-2-phenyl-äthyl)-piperidin.
256. 2-(2-p-Cyclopentyloxy-2-phenyl-äthyl)-piperidin.
257. 2-(2-o-Cyclohexyloxy-2-phenyl-äthyl)-piperidin.
258. 2-(2-m-Cyclohexyloxy-2-phenyl-äthyl)-piperidin.
259. 2-(2-p-Cyclohexyloxy-2-phenyl-äthyl)-piperidin.
260. 2-(2-o-Trifluormethylphenoxy-2-phenyl-äthyl)-piperidin.
261. 2-(2-m-Trifluormethylphenoxy-2-phenyl-äthyl)-piperidin.
262. 2-(2-p-Trifluormethylphenoxy-2-phenyl-äthyl)-piperidin.
263. 2-(2-o-Cyanphenoxy-2-phenyl-äthyl)-piperidin.
264. 2-(2-m-Cyanphenoxy-2-phenyl-äthyl)-piperidin.
265. 2-(2-p-Cyanphenoxy-2-phenyl-äthyl)-piperidin.
266. 2-(2-o-Methylthiophenoxy-2-phenyl-äthyl)-piperidin.
267. 2-(2-m-Methylthiophenoxy-2-phenyl-äthyl)-piperidin.
268. 2-(2-p-Methylthiophenoxy-2-phenyl-äthyl)-piperidin.
269. 2-(2-o-Trifluormethylthiophenoxy-2-phenyl-äthyl)-piperidin.

270. 2-(2-m-Trifluormethylthiophenoxy-2-phenyl-äthyl)-piperidin.

271. 2-(2-p-Trifluormethylthiophenoxy-2-phenyl-äthyl)-piperidin.

272. 2-(2-o-Hydroxyphenoxy-2-phenyl-äthyl)-piperidin.

273. 2-(2-m-Hydroxyphenoxy-2-phenyl-äthyl)-piperidin.

274. 2-(2-p-Hydroxyphenoxy-2-phenyl-äthyl)-piperidin.

275. 2-(2-o-Acetoxyphenoxy-2-phenyl-äthyl)-piperidin.

276. 2-(2-m-Acetoxyphenoxy-2-phenyl-äthyl)-piperidin.

277. 2-(2-p-Acetoxyphenoxy-2-phenyl-äthyl)-piperidin.

278. 2-(2-p-Propionyloxyphenoxy-2-phenyl-äthyl)-piperidin.

279. 2-(2-p-Butyryloxyphenoxy-2-phenyl-äthyl)-piperidin.

280. 2-(2-p-Valeryloxyphenoxy-2-phenyl-äthyl)-piperidin.

281. 2-(2-p-Hexanoyloxyphenoxy-2-phenyl-äthyl)-piperidin.

282. 2-(2-p-Heptanoyloxyphenoxy-2-phenyl-äthyl)-piperidin.

283. 2-[2-(3,4-Dimethoxyphenoxy)-2-phenyl-äthyl]-piperidin.

284. 2-[2-(2-Methoxy-5-trifluormethyl-phenoxy)-2-phenyl-äthyl]-piperidin.

285. 2-[2-(2-Hydroxy-4-chlorphenoxy)-2-phenyl-äthyl]-piperidin.

286. 2-[2-(2-Heptanoyloxy-4-chlorphenoxy)-2-phenyl-äthyl]-piperidin.

287. 2-[2-(2-Decanoyloxy-4-chlorphenoxy)-2-phenyl-äthyl]-piperidin.

288. 1-Methyl-2-(2-phenoxy-2-phenyl-äthyl)-piperidin.

289. 1-Methyl-2-(2-o-fluorphenoxy-2-phenyl-äthyl)-piperidin.

290. 1-Methyl-2-(2-m-fluorphenoxy-2-phenyl-äthyl)-piperidin.

291 1-Methyl-2-(2-p-fluorphenoxy-2-phenyl-äthyl)-piperidin, Hydrochlorid, F. 85-86°.

292. 1-Methyl-2-(2-o-chlorphenoxy-2-phenyl-äthyl)-piperidin.

293. 1-Methyl-2-(2-m-chlorphenoxy-2-phenyl-äthyl)-piperidin.

294. 1-Methyl-2-(2-p-chlorphenoxy-2-phenyl-äthyl)-
295. piperidin, Hydrochlorid, F. 88$^{\circ}$.
295. 1-Methyl-2-(2-o-bromphenoxy-2-phenyl-äthyl)-piperidin.
296. 1-Methyl-2-(2-m-bromphenoxy-2-phenyl-äthyl)-piperidin.
297. 1-Methyl-2-(2-p-bromphenoxy-2-phenyl-äthyl)-piperidin.
298. 1-Methyl-2-(2-o-tolyloxy-2-phenyl-äthyl)-piperidin.
299. 1-Methyl-2-(2-m-tolyloxy-2-phenyl-äthyl)-piperidin.
300. 1-Methyl-2-(2-p-tolyloxy-2-phenyl-äthyl)-piperidin, Hydrochlorid, F. 183$^{\circ}$.
301. 1-Methyl-2-(2-o-methoxyphenoxy-2-phenyl-äthyl)-piperidin, Hydrochlorid, F. 158$^{\circ}$.
302. 1-Methyl-2-(2-m-methoxyphenoxy-2-phenyl-äthyl)-piperidin.
303. 1-Methyl-2-(2-p-methoxyphenoxy-2-phenyl-äthyl)-piperidin, Hydrochlorid, F. 188$^{\circ}$.
304. 1-Methyl-2-(2-o-cyclopentyloxy-2-phenyl-äthyl)-piperidin.
305. 1-Methyl-2-(2-m-cyclopentyloxy-2-phenyl-äthyl)-piperidin.
306. 1-Methyl-2-(2-p-cyclopentyloxy-2-phenyl-äthyl)-piperidin.
307. 1-Methyl-2-(2-o-cyclohexyloxy-2-phenyl-äthyl)-piperidin.
308. 1-Methyl-2-(2-m-cyclohexyloxy-2-phenyl-äthyl)-piperidin.
309. 1-Methyl-2-(2-p-cyclohexyloxy-2-phenyl-äthyl)-piperidin.
310. 1-Methyl-2-(2-o-trifluormethylphenoxy-2-phenyl-äthyl)-piperidin.
311. 1-Methyl-2-(2-m-trifluormethylphenoxy-2-phenyl-äthyl)-piperidin.
312. 1-Methyl-2-(2-p-trifluormethylphenoxy-2-phenyl-äthyl)-piperidin.Hydrochlorid, F. 135$^{\circ}$.

313. 1-Methyl-2-(2-o-cyanphenoxy-2-phenyl-äthyl)-piperidin.

314. 1-Methyl-2-(2-m-cyanphenoxy-2-phenyl-äthyl)-piperidin.

315. 1-Methyl-2-(2-p-cyanphenoxy-2-phenyl-äthyl)-piperidin.

316. 1-Methyl-2-(2-o-methylthiophenoxy-2-phenyl-äthyl)-piperidin, Hydrochlorid F. 164°.

317. 1-Methyl-2-(2-m-methylthiophenoxy-2-phenyl-äthyl)-piperidin.

318. 1-Methyl-2-(2-p-methylthiophenoxy-2-phenyl-äthyl)-piperidin, Hydrochlorid, F. 195-196°.

319. 1-Methyl-2-(2-o-trifluormethylthiophenoxy-2-phenyl-äthyl)-piperidin.

320. 1-Methyl-2-(2-m-trifluormethylthiophenoxy-2-phenyl-äthyl)-piperidin.

321. 1-Methyl-2-(2-p-trifluormethylthiophenoxy-2-phenyl-äthyl)-piperidn.

322. 1-Methyl-2-(2-o-hydroxyphenoxy-2-phenyl-äthyl)-piperidin.

323. 1-Methyl-2-(2-m-hydroxyphenoxy-2-phenyl-äthyl)-piperidin.

324. 1-Methyl-2-(2-p-hydroxyphenoxy-2-phenyl-äthyl)-piperidin.

325. 1-Methyl-2-(2-o-acetoxyphenoxy-2-phenyl-äthyl)-piperidin.

326. 1-Methyl-2-(2-m-acetoxyphenoxy-2-phenyl-äthyl)-piperidin.

327. 1-Methyl-2-(2-p-acetoxyphenoxy-2-phenyl-äthyl)-piperidin.

328. 1-Methyl-2-(2-p-propionyloxyphenoxy-2-phenyl-äthyl)-piperidin.

329. 1-Methyl-2-(2-p-butyryloxyphenoxy-2-phenyl-äthyl)-piperidin.

330. 1-Methyl-2-(2-p-valeryloxyphenoxy-2-phenyl-äthyl)-piperidin.

331. 1-Methyl-2-(2-p-hexanoyloxyphenoxy-2-phenyl-äthyl)-piperidin.

332.1-Methyl-2-(2-p-heptanoyloxyphenoxy-2-phenyl-äthyl)-piperidin.

333.1-Methyl-2-/2-(3,4-dimethoxyphenoxy)-2-phenyl-äthyl7-piperidin.

334.1-Methyl-2-/2-(2-methoxy-5-trifluormethyl-phenoxy)-2-phenyl-äthyl7-piperidin.

335.1-Methyl-2-/2-(2-hydroxy-4-chlorphenoxy)-2-phenyl-äthyl7-piperidin.

336.1-Methyl-2-/2-(2-heptanoyloxy-4-chlorphenoxy)-2-phenyl-äthyl7-piperidin.

337.1-Methyl-2-/2-(2-decanoyloxy-4-chlorphenoxy)-2-phenyl-äthyl7-piperidin.

338.3-Methyl-4-phenoxy-hexahydroazepin.

339.3-Methyl-4-o-fluorphenoxy-hexahydroazepin.

340.3-Methyl-4-m-fluorphenoxy-hexahydroazepin.

341.3-Methyl-4-p-fluorphenoxy-hexahydroazepin.

342.3-Methyl-4-o-chlorphenoxy-hexahydroazepin.

343.3-Methyl-4-m-chlorphenoxy-hexahydroazepin.

344.3-Methyl-4-p-chlorphenoxy-hexahydroazepin.

345.3-Methyl-4-o-bromphenoxy-hexahydroazepin.

346.3-Methyl-4-m-bromphenoxy-hexahydroazepin.

347.3-Methyl-4-p-bromphenoxy-hexahydroazepin.

348.3-Methyl-4-o-tolyloxy-hexahydroazepin.

349.3-Methyl-4-m-tolyloxy-hexahydroazepin.

350.3-Methyl-4-p-tolyloxy-hexyhydroazepin.

351.3-Methyl-4-p-äthylphenoxy-hexahydroazepin.

352.3-Methyl-4-p-isobutylphenoxy-hexahydroazepin.

353.3-Methyl-4-o-methoxyphenoxy-hexahydroazepin.

354.3-Methyl-4-m-methoxyphenoxy-hexahydroazepin.

355.3-Methyl-4-p-methoxyphenoxy-hexahydroazepin.

356.3-Methyl-4-p-äthoxyphenoxy-hexahydroazepin.

357.3-Methyl-4-p-sek.-butoxyphenoxy-hexahydroazepin.

358.3-Methyl-4-p-cyclopropyloxyphenoxy-hexahydroazepin.

359.3-Methyl-4-o-cyclopentyloxyphenoxy-hexahydroazepin.

360.3-Methyl-4-m-cyclopentyloxyphenoxy-hexahydroazepin.

361. 3-Methyl-4-p-cyclopentyloxyphenoxy-hexahydroazepin.
362. 3-Methyl-4-o-cyclohexyloxyphenoxy-hexahydroazepin.
363. 3-Methyl-4-m-cyclohexyloxyphenoxy-hexahydroazepin.
364. 3-Methyl-4-p-cyclohexyloxyphenoxy-hexahydroazepin.
365. 3-Methyl-4-o-trifluormethylphenoxy-hexahydroazepin.
366. 3-Methyl-4-m-trifluormethylphenoxy-hexahydroazepin.
367. 3-Methyl-4-p-trifluormethylphenoxy-hexahydroazepin.
368. 3-Methyl-4-o-cyanphenoxy-hexahydroazepin.
369. 3-Methyl-4-m-cyanphenoxy-hexahydroazepin.
370. 3-Methyl-4-p-cyanphenoxy-hexahydroazepin.
371. 3-Methyl-4-o-methylthiophenoxy-hexahydroazepin.
372. 3-Methyl-4-m-methylthiophenoxy-hexahydroazepin.
373. 3-Methyl-4-p-methylthiophenoxy-hexahydroazepin.
734. 3-Methyl-4-p-äthylthiophenoxy-hexahydroazepin.
375. 3-Methyl-4-p-isobutylthiophenoxy-hexahydroazepin.
376. 3-Methyl-4-o-trifluormethylthiophenoxy-hexahydroazepin.
377. 3-Methyl-4-m-trifluormethylthiophenoxy-hexahydroazepin.
378. 3-Methyl-4-p-trifluormethylthiophenoxy-hexahydroazepin.
379. 3-Methyl-4-o-hydroxyphenoxy-hexahydroazepin.
380. 3-Methyl-4-m-hydroxyphenoxy-hexahydroazepin.
381. 3-Methyl-4-p-hydroxyphenoxy-hexahydroazepin.
382. 3-Methyl-4-o-acetoxyphenoxy-hexahydroazepin.
383. 3-Methyl-4-m-acetoxyphenoxy-hexahydroazepin.
384. 3-Methyl-4-p-acetoxyphenoxy-hexahydroazepin.
385. 3-Methyl-4-p-propionyloxyphenoxy-hexahydroazepin.
386. 3-Methyl-4-p-butyryloxyphenoxy-hexahydroazepin.
387. 3-Methyl-4-p-valeryloxyphenoxy-hexahydroazepin.
388. 3-Methyl-4-p-hexanoyloxyphenoxy-hexahydroazepin.
389. 3-Methyl-4-p-heptanoyloxyphenoxy-hexahydroazepin.

390. 3-Methyl-4-(3,4-dimethoxy-phenoxy)-hexahydroazepin.
391. 3-Methyl-4-(2-methoxy-5-trifluormethyl-phenoxy)-hexahydroazepin.
392. 3-Methyl-4-(2-hydroxy-4-chlor-phenoxy)-hexahydroazepin.
393. 3-Methyl-4-(2-heptanoyloxy-4-chlor-phenoxy)-hexahydroazepin.
394. 3-Methyl-4-(2-decanoyloxy-4-chlor-phenoxy)-hexahydroazepin.
395. 1,3-Dimethyl-4-phenoxy-hexahydroazepin.
396. 1,3-Dimethyl-4-o-fluorphenoxy-hexahydroazepin.
397. 1,3-Dimethyl-4-m-fluorphenoxy-hexahydroazepin.
398. 1,3-Dimethyl-4-p-fluorphenoxy-hexahydroazepin, 2 Isomere, Kp. 80-81°/0,03 mm bzw. Kp. 85-86°/0,03 mm.
399. 1,3-Dimethyl-4-o-chlorphenoxy-hexahydroazepin.
400. 1,3-Dimethyl-4-m-chlorphenoxy-hexahydroazepin.
401. 1,3-Dimethyl-4-p-chlorphenoxy-hexahydroazepin.
402. 1,3-Dimethyl-4-o-bromphenoxy-hexahydroazepin.
403. 1,3-Dimethyl-4-m-bromphenoxy-hexahydroazepin.
404. 1,3-Dimethyl-4-p-bromphenoxy-hexahydroazepin.
405. 1,3-Dimethyl-4-o-tolyloxy-hexahydroazepin.
406. 1,3-Dimethyl-4-m-tolyloxy-hexahydroazepin.
407. 1,3-Dimethyl-4-p-tolyloxy-hexahydroazepin.
408. 1,3-Dimethyl-4-p-äthylphenoxy-hexahydroazepin.
409. 1,3-Dimethyl-4-p-isobutylphenoxy-hexahydroazepin.
410. 1,3-Dimethyl-4-o-methoxyphenoxy-hexahydroazepin.
411. 1,3-Dimethyl-4-m-methoxyphenoxy-hexahydroazepin.
412. 1,3-Dimethyl-4-p-methoxyphenoxy-hexahydroazepin, 2 ölige Isomere, Rf 0,36 bzw. 0,55.
413. 1,3-Dimethyl-4-p-äthoxyphenoxy-hexahydroazepin.
414. 1,3-Dimethyl-4-p-sek.-butoxyphenoxy-hexahydroazepin.
415. 1,3-Dimethyl-4-p-cyclopropyloxyphenoxy-hexahydroazepin.
416. 1,3-Dimethyl-4-o-cyclopentyloxyphenoxy-hexahydroazepin.
417. 1,3-Dimethyl-4-m-cyclopentyloxyphenoxy-hexahydroazepin.

418.1,3-Dimethyl-4-p-cyclopentyloxyphenoxy-hexahydroazepin.

419.1,3-Dimethyl-4-o-cyclohexyloxyphenoxy-hexahydroazepin.

420.1,3-Dimethyl-4-m-cyclohexyloxyphenoxy-hexahydroazepin.

421.1,3-Dimethyl-4-p-cyclohexyloxyphenoxy-hexahydroazepin.

422.1,3-Dimethyl-4-o-trifluormethylphenoxy-hexahydroazepin.

423.1,3-Dimethyl-4-m-trifluormethylphenoxy-hexahydroazepin.

424.1,3-Dimethyl-4-p-trifluormethylphenoxy-hexahydroazepin. Rf 0,5 (Toluol/Triäthylamin 8:2).

425.1,3-Dimethyl-4-o-cyanphenoxy-hexahydroazepin.

426.1,3-Dimethyl-4-m-cyanphenoxy-hexahydroazepin.

427.1,3-Dimethyl-4-p-cyanphenoxy-hexahydroazepin.

428.1,3-Dimethyl-4-o-methylthiophenoxy-hexahydroazepin.

429.1,3-Dimethyl-4-m-methylthiophenoxy-hexahydroazepin.

430.1,3-Dimethyl-4-p-methylthiophenoxy-hexahydroazepin, 2 ölige Isomere, Rf 0,50 bzw. 0,31.

431.1,3-Dimethyl-4-p-äthylthiophenoxy-hexahydroazepin.

432.1,3-Dimethyl-4-p-isobutylthiophenoxy-hexahydroazepin.

433.1,3-Dimethyl-4-o-trifluormethylthiophenoxy-hexahydroazepin.

434.1,3-Dimethyl-4-m-trifluormethylthiophenoxy-hexahydroazepin.

435.1,3-Dimethyl-4-p-trifluormethylthiophenoxy-hexahydroazepin.

436.1,3-Dimethyl-4-o-hydroxyphenoxy-hexahydroazepin.

437.1,3-Dimethyl-4-m-hydroxyphenoxy-hexahydroazepin.

438.1,3-Dimethyl-4-p-hydroxyphenoxy-hexahydroazepin.

439.1,3-Dimethyl-4-o-acetoxyphenoxy-hexahydroazepin.

440.1,3-Dimethyl-4-m-acetoxyphenoxy-hexahydroazepin.

441.1,3-Dimethyl-4-p-acetoxyphenoxy-hexahydroazepin.

442.1,3-Dimethyl-4-p-propionyloxyphenoxy-hexahydroazepin.

443. 1,3-Dimethyl-4-p-butyryloxyphenoxy-hexahydroazepin.
444. 1,3-Dimethyl-4-p-valeryloxyphenoxy-hexahydroazepin.
445. 1,3-Dimethyl-4-p-hexanoyloxyphenoxy-hexahydroazepin.
446. 1,3-Dimethyl-4-p-heptanoyloxyphenoxy-hexahydroazepin.
447. 1,3-Dimethyl-4-(3,4-dimethoxy-phenoxy)-hexahydroazepin.
448. 1,3-Dimethyl-4-(2-methoxy-5-trifluormethyl-phenoxy)-hexahydroazepin.
449. 1,3-Dimethyl-4-(2-hydroxy-4-chlor-phenoxy)-hexahydroazepin.
450. 1,3-Dimethyl-4-(2-heptanoyloxy-4-chlor-phenoxy)-hexahydroazepin.
451. 1,3-Dimethyl-4-(2-decanoyloxy-4-chlor-phenoxy)-hexahydroazepin.

Beispiele 452 bis 457

Analog Beispiel 1 erhält man aus p-Chlorphenol mit 1-Äthyl-, 1-n-Butyl-, 1-Vinyl-, 1-Allyl-, 1-Cyclopropylmethyl- oder 1-Benzyl-2-(2-bromäthyl)-piperidin:

452. 1-Äthyl-2-(2-p-chlorphenoxyäthyl)-piperidin.
453. 1-n-Butyl-2-(2-p-chlorphenoxyäthyl)-piperidin.
454. 1-Vinyl-2-(2-p-chlorphenoxyäthyl)-piperidin.
455. 1-Allyl-2-(2-p-chlorphenoxyäthyl)-piperidin, Rf 0,695 ($CHCl_3$/Triäthylamin 8:2).
456. 1-Cyclopropylmethyl-2-(2-p-chlorphenoxyäthyl)-piperidin, Rf 0,735 ($CHCl_3$/Triäthylamin 8:2).
457. 1-Benzyl-2-(2-p-chlorphenoxyäthyl)-piperidin.

Beispiel 458

Zu einer Lösung von 12,8 g p-Chlorphenol in 100 ml $CH_2Cl_2$ gibt man 200 ml 50 %ige wässerige Natronlauge und 1 g Triäthylbenzylammoniumchlorid, tropft unter

Rühren 16,2 g 1-Methyl-2-(2-chloräthyl)-piperidin hinzu und rührt noch eine Stunde nach. Nach üblicher Aufarbeitung erhält man 1-Methyl-2-(2-p-chlorphenoxyäthyl)-piperidin. Hydrochlorid, F. 139-140°.

Beispiel 459

Eine Lösung von 30,3 g 2-(2-m-Trifluormethylphenoxy-äthyl)-pyridin-hydrochlorid /erhältlich durch Reaktion von Natrium-m-trifluormethylphenolat mit 2-(2-Chloräthyl)-pyridin/ in 1l Äthanol wird an 1 g $PtO_2$ bei 20° und 1 at bis zum Stillstand hydriert. Man filtriert, dampft ein und kristallisiert das erhaltene 2-(2-m-Trifluormethylphenoxy-äthyl)-piperidin-hydrochlorid aus Isopropanol um. F. 152-153°.

Beispiel 460

Eine Lösung von 36,1 g 1-Methyl-2-(2-p-benzyloxyphenoxy-äthyl)-piperidin-hydrochlorid /F. 164°; erhältlich aus Na-p-benzyloxyphenolat und 1-Methyl-2-(2-chloräthyl)-piperidin/ in 400 ml Methanol wird an 3g 5 %igem Pd-C bei 20° und 1 at bis zum Stillstand hydriert. Man filtriert, dampft ein und erhält 1-Methyl-2-(2-p-hydroxyphenoxy-äthyl)-piperidinhydrochlorid, F. 182°.

Beispiel 461

Zu 23,9 g 2-(2-p-Chlorphenoxyäthyl)-piperidin tropft man unter Rühren und Kühlen 30g Ameisensäure und anschließend bei 20° 7g 25 %ige Formaldehydlösung. Man erhitzt bis zum Ende der Gasentwicklung auf dem Wasserbad, kühlt ab, gießt auf Eis, arbeitet wie üblich auf und erhält 1-Methyl-2-(2-p-chlorphenoxyäthyl)-piperidin. Hydrochlorid, F. 139-140°.

Beispiel 462

Man gibt 12 g Allylbromid und 26 g wasserfreies Kaliumcarbonat zu einer Lösung von 23,9 g 2-(2-p-Chlorphenoxyäthyl)-piperidin in 1 l absolutem Toluol, kocht 20 Stunden, kühlt ab, gießt in Wasser, arbeitet wie üblich auf und erhält 1-Allyl-2-(2-p-chlorphenoxyäthyl)-piperidin. Rf 0,7 ($CHCl_3$/Triäthylamin 8:2).

Beispiele 463 bis 466

Analog Beispiel 462 erhält man mit n-Butyljodid, Cyclopropylmethylchlorid oder Benzylbromid:

463. 1-n-Butyl-2-(2-p-chlorphenoxyäthyl)-piperidin.
464. 1-Cyclopropylmethyl-2-(2-p-chlorphenoxyäthyl)-piperidin. Rf 0,5 ($CHCl_3$/ Triäthylamin 8:2).
465. 1-Cyclopropylmethyl-2-(2-p-fluorphenoxyäthyl)-piperidin. Rf 0,5 (Toluol/Triäthylamin 8:2).
466. 1-Benzyl-2-(2-p-chlorphenoxyäthyl)-piperidin.

Beispiel 467

Analog Beispiel 460 erhält man durch Hydrogenolyse von 1-Benzyl-2-(2-p-chlorphenoxyäthyl)-piperidin das 2-(2-p-Chlorphenoxyäthyl)-piperidin, Hydrochlorid, F. 220°.

Beispiel 468

Man löst 23,5 g 1,3-Dimethyl-4-p-hydroxyphenoxy-hexahydroazepin in 100 ml absolutem Aethanol, gibt 200 ml 0,5 n äthanolisches KOH zu, rührt eine Stunde bei 20°, dampft ein und nimmt in 250 ml absolutem DMF auf. Portionsweise wird unter Rühren mit 12,6 g Dimethylsulfat versetzt. Man kocht das Gemisch 2 Stunden, dampft ein, arbeitet wie üblich auf und erhält 1,3-Dimethyl-4-p-methoxyphenoxy-hexahydroazepin, 2 ölige Isomere, Rf 0,36 bzw. 0,55.

Beispiel 469

Man löst 13 g Heptansäure in 10 ml trockenem DMF, gibt 16,2 g Carbonyldiimidazol, dann eine Lösung von 27 g 1-Methyl-2-/2-(2-hydroxy-4-chlorphenoxy)-äthyl/-piperidin in 50 ml trockenem THF und rührt über Nacht bei 20°. Es wird eingedampft und der Rückstand mit ätherischer Salzsäure behandelt. Das ausgefallene 1-Methyl-2-/2-(2-heptanoyloxy-4-chlorphenoxy)-äthyl/-piperidin-hydrochlorid wird abfiltriert und getrocknet.

Die nachstehenden Beispiele betreffen pharmazeutische Zubereitungen, die Amine der Formel I oder ihre Säureadditionssalze enthalten:

Beispiel A: Tabletten

Ein Gemisch von 1 kg 1-Methyl-2-(2-p-chlorphenoxyäthyl)-piperidin-hydrochlorid, 4 kg Lactose, 1,2 kg Kartoffelstärke, 0,2 kg Talk und 0,1 kg Magnesiumstearat wird in üblicher Weise zu Tabletten gepreßt, derart, daß jede Tablette 10 mg Wirkstoff enthält.

Beispiel B: Dragees

Analog Beispiel A werden Tabletten gepreßt, die anschließend in üblicher Weise mit einem Überzug aus Saccharose, Kartoffelstärke, Talk, Tragant und Farbstoff überzogen werden.

Beispiel C: Kapseln

2 kg 1-Methyl-2-(2-p-chlorphenoxyäthyl)-piperidin-hydrochlorid werden in üblicher Weise in Hartgelatinekapseln gefüllt, so daß jede Kapsel 20 mg des Wirkstoffs enthält.

Beispiel D: Ampullen

Eine Lösung von 1 kg 1-Methyl-2-(2-p-chlorphenoxyäthyl)-piperidin-hydrochlorid in 30 l zweifach destilliertem Wasser wird steril filtriert, in Ampullen abgefüllt, unter sterilen Bedingungen lyophilisiert und steril verschlossen. Jede Ampulle enthält 10 mg Wirkstoff.

Analog sind Tabletten, Dragees, Kapseln und Ampullen erhältlich, die einen oder mehrere der übrigen Wirkstoffe der Formel I und/oder ihre physiologisch unbedenklichen Säureadditionssalze enthalten.

Merck Patent Gesellschaft
mit beschränkter Haftung
D a r m s t a d t

Patentansprüche:

1. Phenoxyalkylamine der allgemeinen Formel I

$$Ar\text{-}O\text{-}R \qquad I$$

worin

Ar Phenyl oder ein- oder zweifach durch F, Cl, Br, Alkyl oder Alkoxy mit jeweils 1 - 4 C-Atomen, Cycloalkoxy mit 3 - 6 C-Atomen, $CF_3$, CN, Alkylthio mit 1 - 4 C-Atomen, $SCF_3$, OH und/oder Alkanoyloxy mit 1 - 10 C-Atomen substituiertes Phenyl,

R (1-$R^1$-2-pyrrolidyl)-$CH_2$-$CHR^2$-, (1-$R^1$-2-piperidyl)-$CH_2$-$CHR^2$- oder 1-$R^1$-3-Z-4-hexahydroazepinyl,

$R^1$ H, Alkyl oder Alkenyl mit jeweils bis zu 4 C-Atomen, Cyclopropylmethyl oder Benzyl,

$R^2$ H, Alkyl mit 1 - 4 C-Atomen oder Phenyl und

Z Alkyl mit 1 - 4 C-Atomen

bedeuten, worin jedoch Ar nur dann eine p-Fluorphenylgruppe bedeutet, wenn nicht gleichzeitig R eine 2-(1-Methyl-2-piperidyl)-äthylgruppe ist, sowie deren physiologisch unbedenkliche Säureadditionssalze.

2. 1-Methyl-2-(2-p-chlorphenoxyäthyl)-piperidin.

3. a) 1,3-Dimethyl-4-p-methylthiophenoxy-hexahydroazepin;
   b) 1,3-Dimethyl-4-p-fluorphenoxy-hexahydroazepin;
   c) 1,3-Dimethyl-4-p-methoxyphenoxy-hexahydroazepin;
   d) 1-Methyl-2-(2-p-methylthiophenoxyäthyl)-piperidin;
   e) 2-(2-p-Methylthiophenoxyäthyl)-piperidin;
   f) 1-Methyl-2-(2-m-trifluormethylphenoxyäthyl)-pyrrolidin.

4. Verfahren zur Herstellung von Phenoxyalkylaminen der allgemeinen Formel I

Ar-O-R I

worin

Ar Phenyl oder ein- oder zweifach durch F, Cl, Br, Alkyl oder Alkoxy mit jeweils 1 - 4 C-Atomen, Cycloalkoxy mit 3 - 6 C-Atomen, $CF_3$, CN, Alkylthio mit 1 - 4 C-Atomen, $SCF_3$, OH und/oder Alkanoyloxy mit 1 - 10 C-Atomen substituiertes Phenyl,

R (1-$R^1$-2-pyrrolidyl)-$CH_2$-$CHR^2$-, (1-$R^1$-2-piperidyl)-$CH_2$-$CHR^2$- oder 1-$R^1$-3-Z-4-hexahydroazepinyl,

$R^1$ H, Alkyl oder Alkenyl mit jeweils bis zu 4 C-Atomen, Cyclopropylmethyl oder Benzyl

$R^2$ H, Alkyl mit 1 - 4 C-Atomen oder Phenyl und

Z Alkyl mit 1 - 4 C-Atomen

bedeuten, worin jedoch Ar nur dann eine p-Fluorphenylgruppe bedeutet, wenn nicht gleichzeitig R eine 2-(1-Methyl-2-piperidyl)-äthylgruppe ist,
sowie von deren physiologisch unbedenklichen Säureadditionssalzen, dadurch gekennzeichnet, daß man ein Phenol der allgemeinen Formel II

Ar-OH II

worin

Ar die angegebene Bedeutung hat,

oder eines seiner Salze mit einer Verbindung der allgemeinen Formel III

X-R III

worin

X Cl, Br, J oder OH bedeutet und

R die angegebene Bedeutung hat

oder mit einem ihrer reaktionsfähigen Derivate umsetzt

oder daß man eine Verbindung der allgemeinen Formel IV

$$Ar^1-O-R^3 \qquad IV$$

worin

$Ar^1$ Ar oder einen zu Ar reduzierbaren Rest und

$R^3$ R oder einen zu R reduzierbaren Rest bedeuten und

Ar und R die angegebenen Bedeutungen haben, worin jedoch nicht gleichzeitig $Ar^1$ = Ar und $R^3$ = R sein dürfen,

mit einem Reduktionsmittel behandelt und daß man gegebenenfalls in einer erhaltenen Verbindung der Formel I eine sekundäre Aminogruppe durch Behandeln mit einem alkylierenden, alkenylierenden, cyclopropylmethylierenden oder benzylierenden Mittel in die entsprechende tertiäre Aminogruppe oder eine N-Benzylgruppe durch Behandeln mit einem Reduktionsmittel in eine NH-Gruppe und/oder eine phenolische Hydroxygruppe durch Behandeln mit einem alkylierenden, cycloalkylierenden oder alkanoylierenden Mittel in die entsprechende Alkoxy-, Cycloalkoxy- oder Alkanoyloxygruppe umwandelt und/oder eine erhaltene Base der Formel I durch Behandeln mit einer Säure in eines ihrer physiologisch unbedenklichen Säureadditionssalze umwandelt.

5. Verfahren zur Herstellung pharmazeutischer Zubereitungen, dadurch gekennzeichnet, daß man eine Verbindung der Formel I und/oder eines ihrer physiologisch unbedenklichen Säureadditionssalze zusammen mit mindestens einem festen, flüssigen oder halbflüssigen Träger- oder Hilfsstoff und gegebenenfalls in Kombination mit einem oder mehreren weiteren Wirkstoffen in eine geeignete Dosierungsform bringt.

6. Pharmazeutische Zubereitung, gekennzeichnet durch einen Gehalt an einer Verbindung der allgemeinen Formel I und/oder eines ihrer physiologisch unbedenklichen Säureadditionssalze.